# EUROPEAN PATENT APPLICATION

(11) **EP 2 685 245 A1**
(43) Date of publication of application: **15.01.2014**
(21) Application number: 12755344.4
(22) Date of filing: 08.03.2012
(51) Int. Cl.: G01N 23/05, F16C 19/16, F16C 19/52, F16C 41/00, G01M 13/04

(54) **LUBRICANT DISTRIBUTION ACQUISITION DEVICE AND LUBRICANT DISTRIBUTION ACQUISITION METHOD**

(30) Priority: 10.03.2011 JP 2011053437
(71) Applicant: IHI Corporation, Tokyo 135-8710 (JP)
(72) Inventor: MOCHIKI Koh-ichi, Setagaya-ku, Tokyo 158-8557 (JP); NOSE Hiroyuki, Koto-ku, Tokyo 135-8710 (JP); ITO Akira, Koto-ku, Tokyo 135-8710 (JP); TAKANO Takehisa, Koto-ku, Tokyo 135-8710 (JP)
(74) Representative: Lamb, Martin John Carstairs
(86) International application number: PCT/JP2012/055989
(87) International publication number: WO 2012/121339

(57) **Abstract**

In this lubricant distribution acquisition device (1), neutron beams (L1) that have been transmitted through a bearing (X) from the direction of the main axis thereof or from an oblique direction relative to the main axis thereof are converted into electromagnetic waves, and, by forming images using the received electromagnetic waves, lubricant distribution data that shows the distribution of a lubricant inside the bearing is acquired. As a result, it is possible to ascertain in detail the behavior of the lubricant inside the bearing without dismantling the bearing.

## Description

### [Field of the Invention]

The present invention relates to a lubricant distribution acquisition device and a lubricant distribution acquisition method.

Priority is claimed on Japanese Patent Application No. 2011-53437, filed March 10, 2011, the contents of which are incorporated herein by reference.

### [Background Art]

For example, in Patent document 1, an invention is disclosed that uses neutron radiography to examine whether or not a lubricant is present inside a hydrodynamic bearing.

By using an invention of the type that is disclosed in Patent document 1, without dismantling the bearing it has become possible to perform an examination to determine whether or not a lubricant is present which hitherto has required the bearing to be dismantled.

### [Documents of the prior art]

### [Patent documents]

[Patent document 1] Japanese Patent Application, First Publication No. 2000-292373

### [Disclosure of the Invention]

### [Problems to be Solved by the Invention]

However, although the invention disclosed in Patent document 1 makes it possible to detect whether or not a lubricant is present, it does not enable the way in which the lubricant is distributed over the entire inside of the bearing to be ascertained, and it has not been possible to discover the detailed behavior of the lubricant.

The present invention was conceived in view of the above-described drawback, and it is an object thereof to provide a lubricant distribution acquisition device and a lubricant distribution acquisition method that make it possible to ascertain in detail the behavior of a lubricant inside a bearing.

### [Means for Solving the Problem]

The applicants for the present invention conducted research into the relationship between the behavior of a lubricant inside a bearing and the lifespan of the bearing. As a result, they discovered that individual differences existed between the lifespans of different bearings even when the environment and the like in which they were used were the same. When these bearings having different lifespans were dismantled and examined, it was found that there were considerable differences in the state of the lubricant present inside them. In a roller bearing, in particular, it was found that the behavior of the lubricant inside the bearing had a huge effect on the lifespan.

This suggests that the lifespan of a bearing depends on the behavior of the lubricant inside it. Namely, if the behavior of the lubricant inside a bearing can be ascertained, then there is a possibility that the lifespan of the bearing may be able to be improved.

Based on these research results, a first aspect of the present invention employs a constitution in which a lubricant distribution acquisition device is provided with: an electromagnetic wave converting means that receives neutron beams that have been transmitted through a bearing from the direction of the main axis thereof or from an oblique direction relative to the main axis thereof, and then converts these neutron beams into electromagnetic waves; and an imaging processing means that, by receiving the electromagnetic waves emitted from the electromagnetic wave converting device and using these electromagnetic waves to form images, acquires lubricant distribution data that shows the distribution of a lubricant inside the bearing.

A second aspect of the present invention is the above-described first aspect of the present invention wherein a constitution is employed in which there are provided: a rotation drive means that drives the bearing to rotate; and a protective component that blocks the neutron beams and covers at least a portion of the rotation drive means.

A third aspect of the present invention is the above-described first or second aspects of the present invention wherein a constitution is employed in which there is provided a shielding component that blocks the neutron beams that have not been transmitted through the bearing, and blocks the electromagnetic waves that are created when the neutron beams that have not been transmitted through the bearing are converted by the electromagnetic wave converting means.

A fourth aspect of the present invention is any one of the above-described first through third aspects of the present invention wherein a constitution is employed in which there are provided: a wheel portion whose diameter is set larger than that of the bearing when viewed from the neutron beam irradiation direction and that is fixed to the bearing, and that is formed from a material through which the neutron beam can be transmitted; a belt-shaped component that is entrained around the wheel portion; and a motive power unit that causes the belt-shaped component to run.

A fifth aspect of the present invention is the above-described fourth aspect of the present invention wherein a constitution is employed in which the wheel portion is formed from an aluminum material.

A sixth aspect of the present invention is any one of the above-described first through fifth aspects of the present invention wherein a constitution is employed in which the imaging processing means acquires: first imaging data that is obtained by receiving the electromagnetic waves that are created when the neutron beams that have been transmitted through a first bearing that contains the lubricant inside it are converted by the electromagnetic wave converting means; and second imaging data that is obtained by receiving the electromagnetic waves that are created when the neutron beams that have been transmitted through a second bearing that does not contain the lubricant inside it are converted by the electromagnetic wave converting means, and then acquires the lubricant distribution data from differences between the first imaging data and the second imaging data.

A seventh aspect of the present invention is a lubricant distribution acquisition method and employs a constitution in which neutron beams that have been transmitted through a bearing from the direction of the main axis thereof or from an oblique direction relative to the main axis thereof are converted into electromagnetic waves, and, by forming images using the received electromagnetic waves, lubricant distribution data that shows the distribution of a lubricant inside the bearing is acquired.

### [Effects of the Invention]

Lubricants are formed from organic matter, and their rate of absorption of neutron beams is higher than that of a bearing. Because of this, neutron beams that have been transmitted through a bearing are considerably attenuated in areas where a lubricant is present. On the other hand, the intensity distribution of the neutron beams is proportional to the intensity distribution of the electromagnetic waves into which the neutron beams are converted.

Accordingly, by irradiating neutron beams onto a bearing from the axial direction thereof or from an oblique direction relative to the main axis thereof, and then acquiring images of the electromagnetic waves into which the neutron beams that are transmitted through the bearing have been converted, it is possible to acquire from the brightness distribution of the image data the distribution of the lubricant in a radial direction centered on the main axis.

Moreover, the amount of attenuation of the neutron beams is proportional to the thickness of the lubricant in areas through which the neutron beams are transmitted. Namely, the greater the thickness of the lubricant in these transmission areas, the greater the amount of attenuation of the neutron beams, and the intensity of the neutron beams in these areas is reduced. In contrast, the intensity distribution of the neutron beams is proportional to the intensity distribution of the electromagnetic waves into which the neutron beams are converted. Accordingly, by irradiating neutron beams onto the bearing from the axial direction thereof or from an oblique direction relative to the main axis thereof, and then acquiring images of the electromagnetic waves into which the neutron beams that are transmitted through the bearing have been converted, it is possible to acquire from the brightness distribution of the image data the thickness distribution of the lubricant in the axial direction.

Moreover, in the present invention, by changing the neutron beams that have been irradiated onto a bearing from the axial direction or from an oblique direction relative to the main axis and have been transmitted through the bearing into electromagnetic waves, and then forming images from these received electromagnetic waves, lubricant distribution data that shows the distribution of lubricant inside the bearing is acquired.

Because of this, according to the present invention, it is possible to acquire lubricant distribution data that includes the distribution of a lubricant in a radial direction centered on the main axis and also includes the thickness distribution of the lubricant in an axial direction without having to dismantle the bearing, and it thereby becomes possible to ascertain in detail the behavior of a lubricant inside a bearing.

### [Brief description of the drawings]

[FIG. 1A] FIG. 1A shows the schematic structure of a lubricant distribution acquisition device according to a first embodiment of the present invention, and is a typical view of a portion of the mechanism thereof.
[FIG. 1B] FIG. 1B shows the schematic structure of a lubricant distribution acquisition device according to the first embodiment of the present invention, and is a block diagram showing a portion of the functions thereof.
[FIG. 2] FIG. 2 is a perspective view of a cutaway model showing the schematic structure of a bearing that is installed in the lubricant distribution acquisition device according to the first embodiment of the present invention.
[FIG. 3A] FIG. 3A shows a variant example of the present invention, and is a typical view showing a portion of the mechanism of a lubricant distribution acquisition device according to a second embodiment of the present invention.
[FIG. 3B] FIG. 3B shows a variant example of the present invention, and is a typical view showing a portion of the mechanism of a lubricant distribution acquisition device according to a third embodiment of the present invention.
[FIG. 4A] FIG. 4A is a cross-sectional plan view showing in enlargement a driveshaft portion that is provided in a lubricant distribution acquisition device according to a fourth embodiment of the present invention, and also shows areas adjacent to this driveshaft portion.
[FIG. 4B] FIG. 4B is a frontal view as seen from the axial direction showing the main driveshaft portion shown in FIG. 4A and also areas adjacent thereto.
[FIG. 5] FIG. 5 is a typical view that includes a bearing that is loaded in a lubricant distribution acquisition device according to a fifth embodiment of the present invention.
[FIG. 6] FIG. 6 is a photograph showing an imaging result from the inside of a bearing according to the present invention.

### [Best Embodiments for Implementing the Invention]

Embodiments of the lubricant distribution acquisition device and lubricant distribution acquisition method of the present invention will now be described with reference made to the drawings. Note that in the following drawings, the scale of each component has been suitably altered in order to make each component a recognizable size.

### (First embodiment)

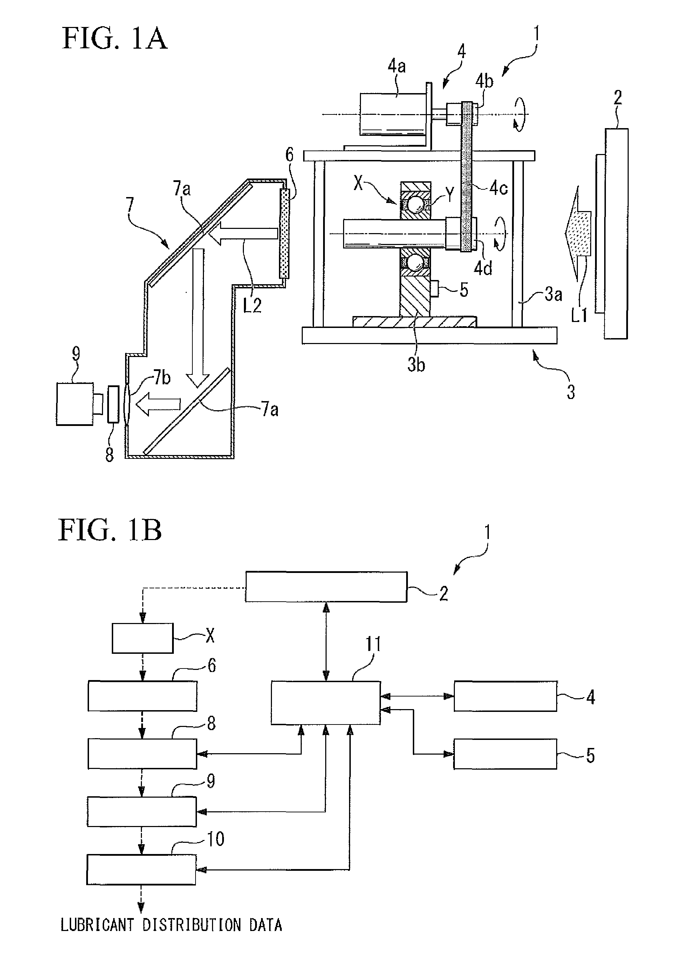

FIGS. 1A and 1B are views showing in typical form the schematic structure of a lubricant distribution acquisition device 1 of the present embodiment. FIG. 1A is a typical view showing a portion of the mechanism of the lubricant distribution acquisition device 1, while FIG. 1B is a block diagram showing a portion of the functions of the lubricant distribution acquisition device 1.

The lubricant distribution acquisition device 1 of the present embodiment ascertains the behavior of a lubricant Y (for example, grease) during the rotation of a bearing X, which is a ball bearing, by acquiring the distribution of the lubricant Y inside the bearing X.

In addition, as is shown in FIGS. 1A and 1B, the lubricant distribution acquisition device 1 of the present embodiment is provided with a neutron beam irradiation device 2, a bearing support mechanism 3, a rotation drive device 4 (i.e., a rotation drive means), a rotation detector 5, a scintillator 6 (i.e., an electromagnetic wave converting means), a light guide mechanism 7, a light amplifier 8, an imaging device 9, a signal processing section 10, and a control unit 11.

The neutron beam irradiation device 2 guides neutron beams L1 emitted from a neutron source such as, for example, an atomic reactor so as to irradiate them onto the bearing X from an axial direction.

Note that if it is possible to irradiate neutron beams emitted from the neutron source onto the bearing X from an axial direction without having to guide the neutron beams, then it is also possible to omit the neutron beam irradiation device 2.

Moreover, in the lubricant distribution acquisition device 1 of the present embodiment, it is also possible to provide a separate neutron source that generates neutron beams by irradiating ions of hydrogen or helium or the like that have been generated by an ion generator, for example, onto a target.

The bearing support mechanism 3 is used to support the bearing X, and is provided with a case body 3a and with a housing 3b.

The case body 3a is a frame body or box-shaped component that contains inside it the housing 3b and the bearing X that is fixed to the housing 3b. In the present embodiment, as is shown in FIG. 1A, the case body 3a also functions as a support base for the rotation drive device 4.

The housing 3b is used to cover and support the outer wheel of the bearing X, and supports the bearing X such that the bearing X can be removably connected thereto. In addition, in the present embodiment, as is shown in FIG. 1A, the housing 3b supports the bearing X such that the main axis of the bearing X faces towards the neutron beam irradiation device 2 side.

Note that it is preferable for the case body 3a and the housing 3b to be shaped such that they avoid the transmission area of the neutron beam L1, however, if they are formed from an aluminum material or the like that has an extremely low neutron beam L1 absorption rate, then the case body 3 a and the housing 3b may be shaped such that they span across the transmission area of the neutron beam L1.

The rotation drive device 4 is used to drive the bearing X to rotate and, as is shown in FIG. 1A, is provided with a motor 4a (i.e., a motive power unit) that generates motive power for driving the bearing X to rotate, a pulley 4b that is used to transmit the motive power generated by the motor 4a to the bearing X by means of a belt, a belt 4c (i.e., a belt-shaped component), and a driveshaft portion 4d.

More specifically, the pulley 4b is joined by a coupling or the like to a shaft portion of the motor 4a. The driveshaft portion 4d is a rod-shaped component that is elongated in the axial direction of the bearing X. The driveshaft portion 4d is fixed to the inner ring of the bearing X, and is placed horizontally so as to penetrate the center of the bearing X. The belt 4c is formed like an endless belt, and is entrained between the pulley 4b and the driveshaft portion 4d.

Note that marks or magnetic bodies that are used by the rotation detector 5 to detect the state of rotation are provided on the circumferential surface of the driveshaft portion 4d.

The rotation detector 5 detects the rotation of the inner ring of the bearing X (namely, detects the rotation of the bearing X) that is fixed to the driveshaft portion 4d by detecting the rotation of the driveshaft portion 4d.

This rotation detector 5 is formed by an optical detector or a magnetic detector that detects the marks or magnetic bodies that are provided on the circumferential surface of the driveshaft portion 4d and, as is shown in FIG. 1A, is fixed to the housing 3b.

The scintillator 6 is used to receive the neutron beam L1 that is transmitted through the bearing X and then emit light L2, and converts the neutron beam L1 into visible light.

For example, LiF/ZnS (Ag), BN/ZnS (Ag), Gd₂O₃/ZnS (Ag), Gd₂O₃S(Tb) can be used for the scintillator 6.

The light guide mechanism 7 guides the light L2 emitted from the scintillator 6 to the imaging device 9 via the light amplifier 8.

As is shown in FIG. 1A, the light guide mechanism 7 is provided with a mirror 7a that reflects and guides the light L2, and with a lens 7b that condenses the light L2.

The light amplifier 8 is used to raise the intensity of the light that enters into it via the light guide mechanism 7, and to then output this light. For example, an image intensifier can be used for the light amplifier 8.

Note that if a sufficiently long exposure time can be guaranteed in the imaging device 9, then it is possible for the optical amplifier 8 to be omitted.

The imaging device 9 is used to receive the light L2 that was emitted from the scintillator 6 and that arrived via the light guide mechanism 7 and the light amplifier 8, and then forms an image using this light. The imaging device 9 outputs the result of this imaging as imaging data.

Note that although a CCD camera, an SIT tube camera, or a high-speed camera or the like can be used for the imaging device 9, because the movement of the lubricant Y inside the bearing X that is rotating at, for example, approximately 6000 rpm is extremely fast, it is preferable for a high-speed camera that is capable of obtaining images at an extremely high frame rate of approximately 2000 fps to be used.

The signal processing section 10 processes imaging data input from the imaging device 9, and outputs it as requested lubricant distribution data.

The lubricant distribution data referred to here is data that includes information pertaining to the distribution of a lubricant in a radial direction centered on the main axis, and information pertaining to the thickness distribution of the lubricant in an axial direction. The signal processing section 10 of the present embodiment, for example, calculates lubricant distribution data from the brightness information in the imaging data, and performs processing to associate this lubricant distribution data with the detection results from the rotation detector 5.

Note that because the information pertaining to the distribution of a lubricant in a radial direction centered on the main axis, and information pertaining to the thickness distribution of the lubricant in an axial direction are contained in the actual imaging data itself that was obtained by the imaging device 9, it is also possible for requested lubricant distribution data to be in the form of imaging data. In this case, the signal processing section 10 outputs the imaging data input from the imaging device 9 as lubricant distribution data without modifying it in any way.

Note that in the present embodiment, an imaging processing means of the present invention is formed by both the imaging device 9 and the signal processing section 10.

The control unit 11 is used to control the overall operations of the lubricant distribution acquisition device 1 of the present embodiment and, as is shown in FIG. 1B, the control unit 11 is electrically connected to the neutron beam irradiation device 2, the rotation drive device 4, the rotation detector 5, the light amplifier 8, the imaging device 9, and the signal processing section 10.

The bearing X is a ball bearing (i.e., a roller bearing) that contains a lubricant inside it and, in the present embodiment, is formed as a radial bearing.

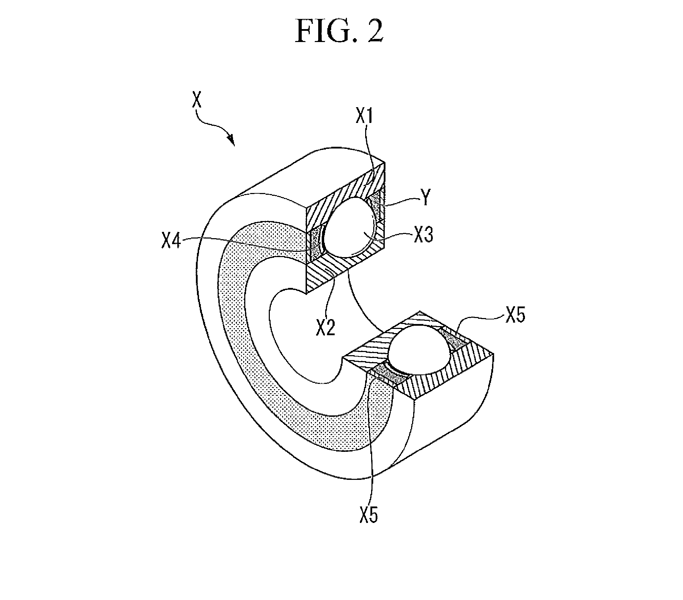

FIG. 2 is a perspective view of a cutaway model showing the schematic structure of the bearing X. As is shown in this drawing, the bearing X is provided with a toroidal outer ring X1 and a toroidal inner ring X2 that are positioned facing each other in a radial direction, a plurality of balls X3 that are located between the outer ring X1 and the inner ring X2, a holder X4 that is used to maintain equidistant intervals between adjacent balls X3, and seals X5 that seal off the spaces where the balls X3 are housed.

Note that in order to raise the visibility of the lubricant Y in the imaging data and to thereby acquire a more accurate distribution, it is desirable that component elements of the bearing X do not appear in the imaging data. Because of this, it is preferable for these component elements of the bearing X (i.e., the outer ring X1, the inner ring X2, the balls X3, the holder X4, and the seals X5) to be formed from an aluminum material that has a low absorption rate of the neutron beam L1.

Next, operations (i.e., a lubricant distribution acquisition method) of the lubricant distribution acquisition device 1 of the present embodiment which is constructed in the manner described above will be described. Note that the main agent of the operations of the lubricant distribution acquisition device 1 of the present embodiment that are described below is the control unit 11.

Firstly, the control unit 11 causes the bearing X to be rotated by the rotation drive device 4. As a result of this, the inner ring X2 of the bearing X is driven to rotate, and the balls X3 that are sandwiched between the inner ring X2 and the outer ring X1 revolve around the main axis at the same time as they are rotated around their own axis. As a consequence, the lubricant Y moves through the interior of the bearing X in conjunction with the movement of the balls X3.

Next, the neutron beam L1 from the neutron beam irradiation device 2 is guided to the bearing X side. As a result of this, as is shown in FIG. 1A, the neutron beam L1 enters into the bearing X from the axial direction of the bearing X, and the neutron beam L1 that is transmitted through the bearing X then enters into the scintillator 6.

When the neutron beam L1 enters into the scintillator 6, the scintillator 6 emits light L2 that has the same intensity distribution as the intensity distribution of the neutron beam L1. Namely, the scintillator 6 converts the neutron beam L1 into the light L2 and then emits this light L2.

The light L2 emitted from the scintillator 6 is guided by the light guide mechanism 7 and amplified by the light amplifier 8, and then enters into the imaging device 9.

The control unit 11 then causes the imaging device 9 to create an image. As a result of this, imaging data is acquired by the imaging device 9.

Next, the control unit 11 causes the signal processing section 10 to process the imaging data, and to also calculate lubricant distribution data that includes information pertaining to the distribution of the lubricant in a radial direction centered on the main axis, and information pertaining to the thickness distribution of the lubricant in the axial direction.

The control unit 11 also performs processing to associate the calculated lubricant distribution data with the detection results from the rotation detector 5. As a result of this, the lubricant distribution data is output in association with the rotation angle of the bearing X.

Here, the lubricant Y is formed from an organic material so that it has a higher rate of neutron beam absorption than does the bearing X. Because of this, the neutron beam L1 that has been transmitted through the bearing X is greatly attenuated in areas where the lubricant Y is present. In contrast, the intensity distribution of neutron beam L1 is proportional to the intensity distribution of the light L2 into which the neutron beam L1 has been converted.

Accordingly, by irradiating the neutron beam L1 onto the bearing X from the axial direction, and then acquiring images of the light L2 into which the neutron beam L1 that is transmitted through the bearing X has been converted, it is possible to acquire from the brightness distribution of the image data the distribution of the lubricant Y in a radial direction centered on the main axis.

Moreover, the amount of attenuation of the neutron beam L1 is proportional to the thickness of the lubricant Y in areas through which the neutron beam L1 is transmitted. Namely, the greater the thickness of the lubricant Y in these transmission areas, the greater the amount of attenuation of the neutron beam L1, and the intensity of the neutron beam L after being transmitted through these areas is reduced. In contrast, the intensity distribution of the neutron beam L1 is proportional to the intensity distribution of the light L2 into which the neutron beam L1 is converted. Accordingly, by irradiating the neutron beam L1 onto the bearing X from the axial direction, and then acquiring images of the light L2 into which the neutron beam L1 that is transmitted through the bearing X has been converted, it is possible to acquire from the brightness distribution of the image data the thickness distribution of the lubricant in the axial direction.

In addition, in the lubricant distribution acquisition device 1 and the lubricant distribution acquisition method of the present embodiment, by changing the neutron beam L1 that has been irradiated onto the bearing X from the axial direction and has been transmitted through the bearing X into the light L2, and then forming images from the received light L2, lubricant distribution data that shows the distribution of the lubricant Y inside the bearing X is acquired.

Because of this, according to the lubricant distribution acquisition device 1 and the lubricant distribution acquisition method of the present embodiment, it is possible to acquire lubricant distribution data that includes the distribution of the lubricant Y in a radial direction centered on the main axis and also includes the thickness distribution of the lubricant Y in an axial direction without having to dismantle the bearing X, and it thereby becomes possible to ascertain in detail the behavior of the lubricant Y inside the bearing X.

### (Second embodiment)

Next, a second embodiment of the present invention will be described. Note that in the description of the second embodiment, any description of portions thereof that are the same as those of the first embodiment is either omitted or simplified.

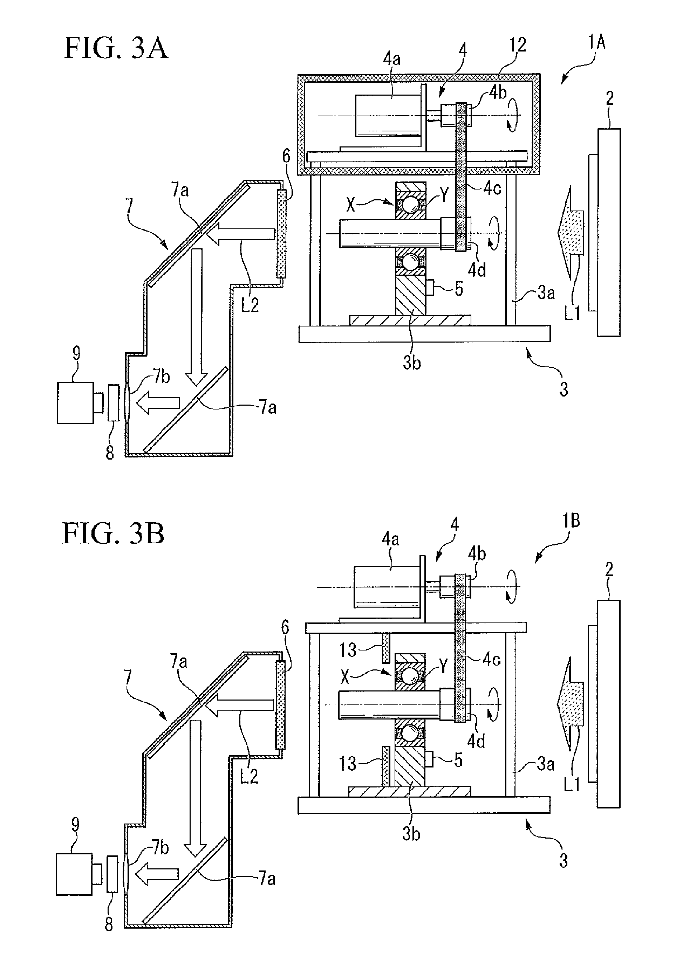

FIG. 3A is a typical view showing a portion of the mechanism of a lubricant distribution acquisition device 1A of the present embodiment. As is shown in these drawings, the lubricant distribution acquisition device 1A of the present embodiment is provided with a protective box (i.e., a protective component) 12.

This protective box 12 is formed from a material that blocks out neutron beams, and prevents the neutron beam L1 being transmitted into the interior thereof. Specifically, the protective box 12 may be formed from a rubber material containing, for example, boron.

As is shown in FIG. 3A, this protective box 12 is placed on top of the case body 3a of the bearing support mechanism 3 so as to surround a motor 4a which forms a portion of the rotation drive device 4.

According to the lubricant distribution acquisition device 1A of the present embodiment which employs this type of structure, it is possible to prevent neutron beams reaching the motor 4a that is housed inside the protective box 12, so that the motor 4a is prevented from becoming radioactive.

As is commonly known, radioactive components must be controlled in a restricted environment, and the cost of controlling such components is increased. In contrast to this, because it is possible to prevent the motor 4a from becoming radioactive in the lubricant distribution acquisition device 1A of the present embodiment, it is possible to suppress any increase in the control costs.

Note that it is not essential for the protective material of the present invention to be formed in a box shape. Because of this, it is also possible to fit a plate-shaped protective component instead of the protective box 12 such that the neutron beam L1 is not irradiated onto the motor 4a.

### (Third embodiment)

Next, a third embodiment of the present invention will be described. Note that in the description of the third embodiment as well, any description of portions thereof that are the same as those of the first embodiment is either omitted or simplified.

FIG. 3B is a typical view showing a portion of the mechanism of a lubricant distribution acquisition device 1B of the present embodiment. As is shown in this drawing, the lubricant distribution acquisition device 1B of the present embodiment is provided with a shielding wall (i.e., a shielding component) 13.

The shielding wall 13 blocks neutron beams that have not been transmitted through the bearing X, and prevents the neutron beam L1 from being transmitted to the scintillator 6 side. As is shown in FIG. 3B in the present embodiment, the shielding wall 13 is positioned between the bearing X and the scintillator 6 and, when looking from the axial direction, is placed around the periphery of the bearing X such that it blocks the neutron beam L1 that is being transmitted around the periphery of the bearing X.

This shielding wall 13 can be formed from a rubber material containing, for example, boron.

The neutron beam L1 that is not transmitted through the bearing X is irradiated essentially unattenuated onto the scintillator 6. Consequently, the light L2 that is emitted from the scintillator 6 has an extremely strong intensity. As a result of this, in the imaging data, extremely bright areas exist outside the areas where the lubricant Y is present, and the dynamic range in the areas where the lubricant Y is present is decreased due to the presence of the aforementioned extremely bright areas.

In contrast to this, according to the lubricant distribution acquisition device 1B of the present embodiment, the neutron beam L1 that is not transmitted through the bearing X is blocked by the shielding wall 13. As a consequence, it is possible to prevent extremely bright areas being generated in the imaging data outside the areas where the lubricant Y is present, and it becomes possible to increase the dynamic range in the areas where the lubricant Y is present. As a result, it becomes possible to acquire more detailed lubricant distribution data, and to ascertain in even more detail the behavior of the lubricant Y inside the bearing X.

Note that the location where the shielding component of the present invention is installed is not limited to the installation location of the shielding wall 13 shown in FIG. 3B. In order to increase the dynamic range in areas where the lubricant Y is present, it is sufficient if extremely bright areas are prevented from being generated in the imaging data outside the areas where the lubricant Y is present. Because of this, it is also possible for the shielding wall 13 to be placed between the bearing X and the neutron beam irradiation device 2.

Moreover it is also possible to block the light L2 emitted from the scintillator 6 instead of blocking the neutron beam L1. Namely, it is also possible for the light L2 that is created when the neutron beam L1 that has not been transmitted through the bearing X is converted in the scintillator 6 to be blocked.

### (Fourth embodiment)

Next, a fourth embodiment of the present invention will be described. Note that in the description of the fourth embodiment as well, any description of portions thereof that are the same as those of the first embodiment is either omitted or simplified.

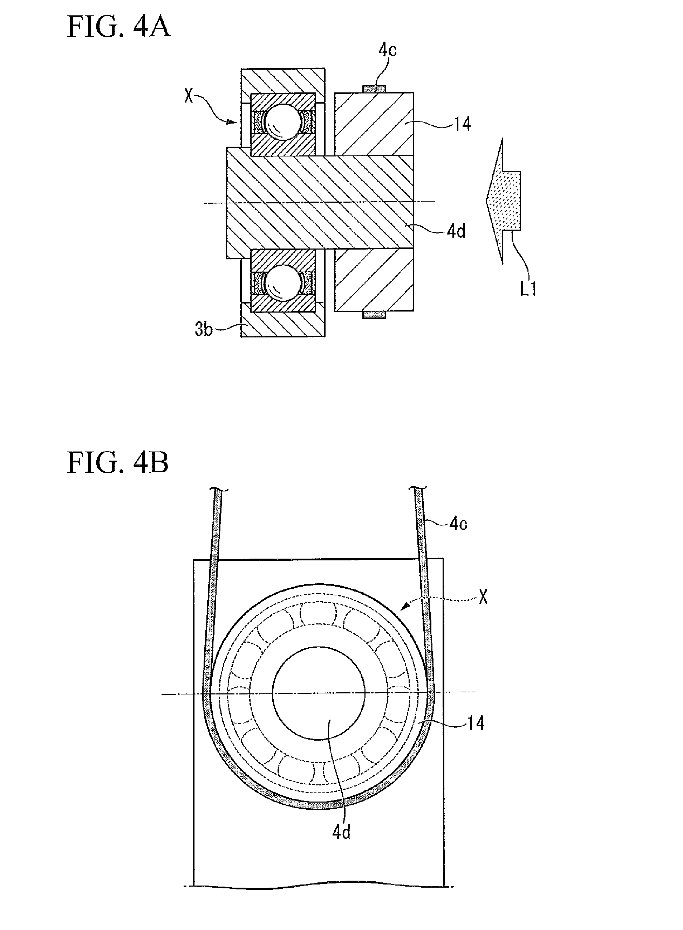

FIGS. 4A and 4B show in enlargement the driveshaft portion 4d that is provided in a lubricant distribution acquisition device of the present embodiment, and also shows areas adjacent to this driveshaft portion with FIG. 4A being a cross-sectional plan view and FIG. 4B being a frontal view as seen from the direction of the main axis.

As is shown in these drawings, the lubricant distribution acquisition device of the present embodiment is provided with a large diameter pulley 14 that is fixed to the bearing X via the driveshaft portion 4d, and whose diameter is set larger than that of the bearing X.

This large diameter pulley 14 is formed from a material that allows a neutron beam to pass through it, and may be formed, for example, from an aluminum material.

In addition, in the lubricant distribution acquisition device of the present embodiment, the belt 4c is entrained around this large diameter pulley 14.

According to the lubricant distribution acquisition device of the present embodiment which has the above-described structure, when viewed from the axial direction, the belt 4c does not overlap with areas where the lubricant Y is present. The belt 4c is formed from a normal rubber material and because it has a high neutron beam absorption rate, it appears as a bright object in the imaging data. Moreover, according to the lubricant distribution acquisition device of the present embodiment, because the belt 4c does not overlap with areas where the lubricant Y is present, it is possible to improve the visibility of the lubricant Y.

Note that as is shown in FIG. 4A, although the large diameter pulley 14 can be formed independently from the driveshaft portion 4d, it may also be formed as a portion of the driveshaft portion 4d.

### (Fifth embodiment)

Next, a fifth embodiment of the present invention will be described. Note that in the description of the fifth embodiment as well, any description of portions thereof that are the same as those of the first embodiment is either omitted or simplified.

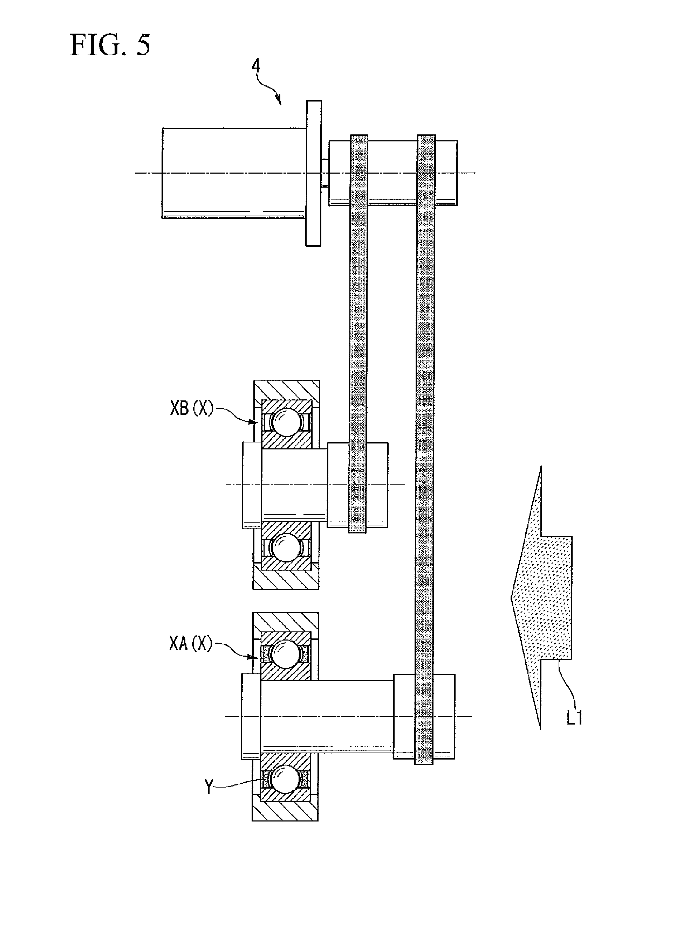

FIG. 5 is a plan view showing a state of a bearing X that is loaded in a lubricant distribution acquisition device of the present embodiment. In the lubricant distribution acquisition device of the present embodiment, a bearing XA (i.e., a first bearing) that has the lubricant Y provided inside it, and a bearing XB (i.e., a second bearing) that does not have the lubricant Y provided inside it are loaded next to each other as the bearing X in the lubricant distribution acquisition device of the present embodiment.

These bearings XA and XB are formed such that they are able to be rotated by the rotation drive device 4.

In the lubricant distribution acquisition device of the present embodiment, in the signal processing section 10, an area among the imaging data in which the bearing XA appears is extracted as first imaging data, and an area among the imaging data in which the bearing XB appears is extracted as second imaging data. The bearing XA and the bearing XB are then superimposed and a difference between the first imaging data and the second imaging data is calculated. Lubricant distribution data is then calculated based on this difference.

According to the lubricant distribution acquisition device of the present embodiment which has the above-described structure, by calculating the difference between the first imaging data and the second imaging data, it is possible to acquire the image data for only the lubricant Y portion, which is formed by the discrepancy portion between the first imaging data and the second imaging data. As a consequence, by using the imaging data for only the lubricant Y portion, it is possible to acquire lubricant distribution data more accurately and in more detail, and it becomes possible to ascertain in even greater detail the behavior of the lubricant Y within the bearing X.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as limited by the foregoing description and is only limited by the scope of the appended claims.

For example, in the rotation drive device it is also possible to use a toothed pulley together with a toothed belt. It is also possible for a sprocket (i.e., a wheel portion) and a chain (i.e., a belt-shaped component) to be used.

Moreover, in the above-described embodiments, a structure in which the bearing X is a ball bearing that receives a load in a radial direction is described.

However, it is also possible for the present invention to be used to ascertain the behavior of a lubricant inside other types of bearing such as, for example, roller bearings, sliding bearings, and bearings that receive a load in a thrust direction.

Moreover, in the above-described embodiments, a structure in which the neutron beam L1 is transmitted through a bearing from an axial direction thereof is described.

However, the present invention is not limited to this and it is also possible for a structure in which the neutron beam L1 is transmitted through the bearing from an oblique direction relative to the main axis to be employed.

Moreover, in the above-described embodiments, a structure in which the neutron beam L1 is converted into light L2 using the scintillator 6 is described.

However, the present invention is not limited to this and it is also possible to acquire images by converting the neutron beam L1 into radioactive rays (i.e., electromagnetic waves) such as gamma rays and the like.

Moreover, in the above-described embodiments, a structure in which digital photography is performed by the imaging device 9 is described.

However, the present invention is not limited to this and it is also possible for film photography to be performed by the imaging device.

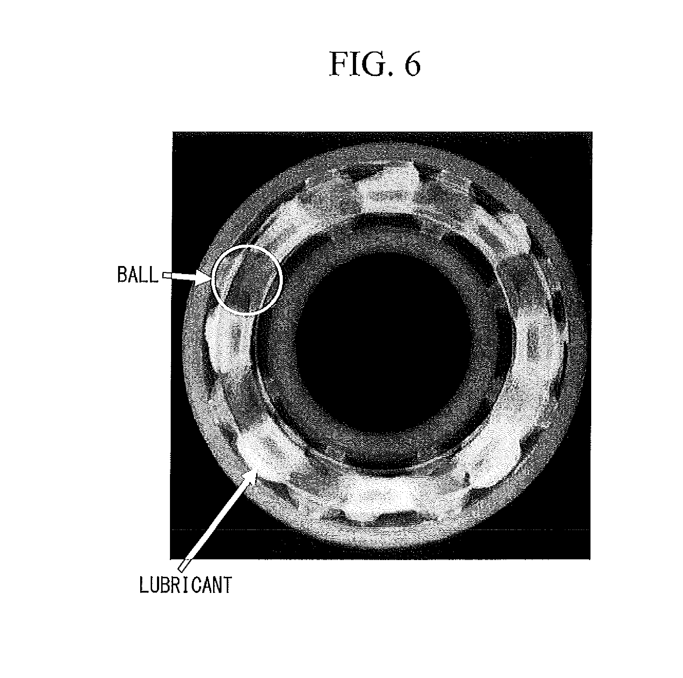

Note that as a result of converting the neutron beam L1 into gamma rays and then performing film photography using the lubricant distribution acquisition device of the present invention, images such as that shown in FIG. 6 were acquired.

As can be understood from this imaging result, according to the present invention, it is possible to acquire an image of the interior of a bearing.

### [Industrial applicability]

According to the present invention, it is possible to ascertain in detail the behavior of a lubricant inside a bearing without dismantling the bearing.

### [Description of the Reference Numerals]

1, 1A, 1B ... Lubricant distribution acquisition device, 2 ... Neutron beam irradiation device, 4a ... Motor (Motive power unit), 4b ... Pulley, 4c ... Belt (Belt-shaped component), 5 ... Rotation detector, 6 ... Scintillator (Electromagnetic wave converting means), 8 ... Light amplifier, 9... Imaging device, 10 ... Signal processing section, 11 ... Control unit, 12 ... Protective box (Protective component), 13 ... Shielding wall (Shielding component), 14 ... Large diameter pulley (Wheel portion), L1 ... Neutron beam, L2 ... Light (Electromagnetic waves), X, XA, XB ... Bearings, Y ... Lubricant

## Claims

1. A lubricant distribution acquisition device comprising:
an electromagnetic wave converting means that receives neutron beams that have been transmitted through a bearing from the direction of the main axis thereof or from an oblique direction relative to the main axis thereof, and then converts these neutron beams into electromagnetic waves; and
an imaging processing means that, by receiving the electromagnetic waves emitted from the electromagnetic wave converting device and using these electromagnetic waves to form images, acquires lubricant distribution data that shows the distribution of a lubricant inside the bearing.

2. The lubricant distribution acquisition device according to claim 1 wherein there are provided:
a rotation drive means that drives the bearing to rotate; and
a protective component that blocks the neutron beams and covers at least a portion of the rotation drive means.

3. The lubricant distribution acquisition device according to claim 1 wherein there is provided a shielding component that blocks the neutron beams that have not been transmitted through the bearing, and blocks the electromagnetic waves that are created when the neutron beams that have not been transmitted through the bearing are converted by the electromagnetic wave converting means.

4. The lubricant distribution acquisition device according to claim 1 wherein there are provided:
a wheel portion whose diameter is set larger than that of the bearing when viewed from the neutron beam irradiation direction and that is fixed to the bearing, and that is formed from a material through which the neutron beam can be transmitted;
a belt-shaped component that is entrained around the wheel portion; and
a motive power unit that causes the belt-shaped component to run.

5. The lubricant distribution acquisition device according to claim 4 wherein the wheel portion is formed from an aluminum material.

6. The lubricant distribution acquisition device according to any one of claims 1 through 5 wherein
the imaging processing means acquires:
first imaging data that is obtained by receiving the electromagnetic waves that are created when the neutron beams that have been transmitted through a first bearing that contains the lubricant inside it are converted by the electromagnetic wave converting means; and
second imaging data that is obtained by receiving the electromagnetic waves that are created when the neutron beams that have been transmitted through a second bearing that does not contain the lubricant inside it are converted by the electromagnetic wave converting means, and
then acquires the lubricant distribution data from differences between the first imaging data and the second imaging data.

7. A lubricant distribution acquisition method in which neutron beams that have been transmitted through a bearing from the direction of the main axis thereof or from an oblique direction relative to the main axis thereof are converted into electromagnetic waves, and, by forming images using the received electromagnetic waves, lubricant distribution data that shows the distribution of a lubricant inside the bearing is acquired.
